# EUROPEAN PATENT SPECIFICATION

(11) **EP 1 858 504 B1**
(45) Date of publication and mention of the grant of the patent: **04.01.2012**
(21) Application number: 06708437.6
(22) Date of filing: 22.02.2006
(51) Int. Cl.: A61K 31/195, A61K 31/35, A61P 13/00, A61M 1/28

(54) **CARNITINE-CONTAINING PERITONEAL DIALYSIS SOLUTION HAVING IMPROVED BIOCOMPATIBILITY**
CARNITIN-HALTIGE PERITONEALDIALYSE-LÖSUNG MIT VERBESSERTER BIOKOMPATIBILITÄT
SOLUTION DE DIALYSE PERITONEALE CONTENANT DE LA CARNITINE ET PRESENTANT UNE BIOCOMPATIBILITE AMELIOREE

(30) Priority: 10.03.2005 WO PCT/IT2005/000130
(43) Date of publication of application: 28.11.2007
(73) Proprietor: SIGMA-TAU Industrie Farmaceutiche Riunite S.p.A., 00144 Roma (IT)
(72) Inventor: ARDUINI, Arduino, 6822 Arogno (CH)
(74) Representative: Spadaro, Marco
(86) International application number: PCT/EP2006/060162
(87) International publication number: WO 2006/094900

(56) References cited:
- WO-A-99/07419
- WO-A-02/081005
- US-B1- 6 822 002
- GAGGIOTTI E ET AL: "Prevention of peritoneal sclerosis: A new proposal to substitute glucose with carnitine dialysis solution (biocompatibility testing in vitro and in rabbits)" INTERNATIONAL JOURNAL OF ARTIFICIAL ORGANS, vol. 28, no. 2, February 2005 (2005-02), pages 177-187, XP009056578 ISSN: 0391-3988
- BAZZATO G ET AL: "XYLITOL AS OSMOTIC AGENT IN CONTINUOUS AMBULATORY PERITONEAL DIALYSIS AN ALTERNATIVE TO GLUCOSE FOR UREMIC DIABETIC PATIENTS?" TRANSACTIONS AMERICAN SOCIETY FOR ARTIFICIAL INTERNAL ORGANS, vol. 28, 1982, pages 280-285, XP009056584 ISSN: 0066-0078

## Description

The present invention relates to the field of peritoneal dialysis, in particular to solutions for peritoneal dialysis having improved biocompatibility.

### Background of the invention

The mesothelium is the first organ to come into contact with dialysis solutions and it tends to gradually lose its viability during peritoneal dialysis. This is evident from morphological changes observed during peritoneal dialysis (Di Paolo, N.; et al., Nephron, 1996; 74:594-9*;* Di Paolo, N.; et al., Perit. Dial. Int., 2000; 20 (Suppl 3): S1 -100). Signs of anatomical distress include a decrease in lubricating capacity and in secretory properties of the peritoneum (Di Paolo, N., et al.; Nephron, 1986; 44, 365-370*;* Shambye, H.T., et al.; Perit. Deal. Intern., , 1992; 12, 284-286*).* To obviate this problem, there is the medical need to find new solutions containing non toxic substances with high osmolarity. The characteristics of the ideal PDS are well known: low glucose, pH 7, good osmotic pressure, free of plasticisers, particles and trace elements and good biocompatibility in vitro and in vivo.

Since the advent of Continuous Ambulatorial Peritoneal Dialysis (CAPD), a valid alternative to the classical PDS has never been marketed. PDS containing bicarbonate, amino acids or icodextrin have had limited clinical success due to systemic side effects (Shambye, H.T., et al.; Perit. Dial. Intern., 1992; 12, 284-286*;* Cooker, L.A., et al.; Kidney Int. Suppl., 2002; (81) S34-45*,* Gotloib, L.; Free Radic. Biol. Med., 2003; Feb 15; 34(4):419-28*;* Holmes, C.J. and Faict, D.; Kidney Int. Suppl., 2003; Dec.; (88):550-6).

The biocompatibility of a peritoneal dialysis solution may be defined as is its capacity to leave the anatomical and functional characteristics of the peritoneum unmodified in time (Di Paolo, N., et al.; Per. Dial. Intern., 1993; 1 (S2), 109-112*;* Di Paolo, N.; et al.; Perit. Dial. Intern., 1995; 15 (supp. 7), 61-70). An important step in the study of biocompatibility of a peritoneal dialysis solution (PDS) is in vitro experimentation, which is conveniently tested in mesothelial cell cultures (Di Paolo, N., et al.; Per. Dial. Intern., 1993; 1 (S2), 109-112*;* Di Paolo, N., et al.; Nephron 1996; 74:594-9*).* According to the above definition, biocompatibility studies in vitro must cover a vast range of aspects from the histological modifications of mesothelial cells to their functional modifications during contact with the solutions (Di Paolo, N., et al.; Perit. Dial. Int., 1994; supp. 3, 12-15).

Peritoneal dialysis solutions must conserve the integrity of the peritoneal membrane and its function, permitting frictionless sliding of the abdominal viscera (Di Paolo, N.; et al.; Nephron, 1986; 44, 365-370*).*

Peritoneal dialysis (PD) exposes the peritoneum to hyperosmolar glucose containing variable amounts of non-enzymic breakdown products of glucose (Di Paolo, N., et al.; Nephron, 1991; 57:323-31*;* Perit. Dial. Int., 2000; 20 (Suppl 3):81-100). These solutions are toxic for the peritoneum. Major ultrastructural changes have been documented in the mesothelium of PD patients. Replacement of glucose with amino acids gave good results (Holmes, C.J.; Perit. Dial. Intern., 1991; 13, 88-94*;* Jorres, A., et al.; Intern. J. of Artif. Organs 1992; 15, 79-83*)* in terms of peritoneal biocompatibility, but clinical application is limited by systemic pharmacological effects due to high absorption (Garosi, G., et al.; Perit. Dial. Intern., 1998; 18: 610-81).

Different proposals for a better biocompatibility of peritoneal dialysis solutions have been presented.

In WO 97/06810, Wu, Tam and French disclose a biocompatible solution for peritoneal dialysis comprising an effective amount of an acetylated or deacetylated aminosugar and/or combinations thereof. These sugars are either monomers or olygomers

US 6,277,815, to Fresenius, discloses a solution for peritoneal dialysis or for infusion comprising two single solutions which are brought together and dispensed, with the first single solution containing calcium ions, additional electrolytic salts and glucose in an osmotically effective concentration and the second single solution containing bicarbonate and a weak acid with pKa<5. To provide a biocompatible solution, in particular for use as a peritoneal dialysis solution, the first single solution is acidified with a physiologically compatible acid to a pH of below 3.2. The second single solution contains bicarbonate only in a proportion which does not exceed 10 mmol/l.

JP 2002282354, to JMS Co. Ltd., provides a biocompatible solution for peritoneal dialysis, in which the absorption of glucose is reduced by adding at least one kind of trehalose or raffinose to the solution.

However, the problem of biocompatibility of solutions for use in peritoneal dialysis is still to be solved in a satisfactory manner. In fact, also osmotic agents alternative to glucose can present unwanted effects or are affected by high production cost, which makes their use difficult in those situations where peritoneal dialysis is practised in socially disadvantaged subjects.

Low L-carnitine biosynthesis may be associated with chronic kidney disease and L-carnitine supplements seem to have beneficial effects in hemodialysed patients (Hurot, J.M., et acl.; J. Am. Soc. Nephrol., 2002; 11, 13:708-14).

WO 01/26649 discloses the use of L-carnitine and its lower alkanoyl derivatives (collectively intended as carnitines) as osmotic agents for medical solutions. In a particular embodiment, carnitines are used as total or partial replacement of glucose in solutions for peritoneal dialysis. A number of combinations of carnitines, in particular L-carnitine and glucose or amino acids in different concentrations are also disclosed. The carnitine is provided in a range of from 0.5% w/v to 10% w/v, being 0.5% and the following specific combinations of L-carnitine and glucose are disclosed: 1.0% w/v L-carnitine and 0.5% w/v glucose; 0.5% w/v L-carnitine and 4.0% w/v glucose; 4.0% w/v L-carnitine and 0.5% w/v glucose.

WO 99/07419, Gupta et al., discloses solutions for dialysis with the aim to provide the patient with a nutritional supplement in order to compensate for the loss of vitamins, which the patients undergoes to during dialysis, either peritoneal or haemodialysis. L-carnitine is foreseen just as an optional ingredient, since vitamins are the main components. Moreover, the concentration range taught by Gupta for L-carnitine is insufficient, even at the highest end (1 mM - 0.016% w/v), to provide an osmotic effect.

The problem of a high biocompatible solution for peritoneal dialysis is still a strong medical need.

It has now surprisingly been found that using L-carnitine or a lower alkanoyl derivatives thereof or a pharmaceutically acceptable salt thereof at a concentration range of about 0.02% w/v to about 0.5%w/v together with an osmotic agent for peritoneal dialysis, in particular glucose, a beneficial effect on biocompatibility of the solution for peritoneal dialysis is obtained.

Another surprising finding is that the addition of xylitol to a solution for peritoneal dialysis containing carnitine and glucose enhances solution biocompatibility.

### Summary of the invention

The present invention is based on the finding that L-carnitine, at a concentration of between about 0.02% w/v to about 0.5% w/v, exerts a protective effect on peritoneum, when exposed to a peritoneal dialysis solution.

Accordingly, it is an object of the present invention a solution for peritoneal dialysis comprising a from 0.02% w/v to 0.5% w/v carnitine and glucose and xylitol for peritoneal dialysis,

Another object of the present invention is the use of such a solution for the preparation of a package for peritoneal dialysis.

These and other objects of the present invention will be disclosed in the detail in the foregoing also by means of examples.

### Detailed description of the invention

Reference is made to WO 01/26649 and the art referred therein for a description of the use of carnitines in peritoneal dialysis and also for the understanding of the present invention in the context of the technical field of peritoneal dialysis.

In the present invention, carnitine means L-carnitine or a lower alkanoyl derivative thereof, L-carnitine is preferred.

A lower alkanoyl derivative is a C₂-C₈ acyl derivative, such as, for example acetyl, propionil, butyryl, isobutyryl, valeryl, isovaleryl, hexanoyl, octanoyl and all their possible isomers.

Normally, carnitine, in particular L-carnitine is used in the form of inner salt. If suitable, a pharmaceutically acceptable salt can be used. Definition and examples of said salts are provided in the above mentioned WO 01/26649.

Examples of pharmaceutically acceptable salts are chloride, bromide, orotate, acid aspartate, acid citrate, magnesium citrate, acid phosphate, fumarate and acid fumarate, magnesium fumarate, lactate, maleate and acid maleate, mucate, acid oxalate, pamoate, acid pamoate, acid sulfate, glucose phosphate, tartrate, acid tartrate, magnesium tartrate, 2-amino ethansulfonate, magnesium 2-amino ethansulfonate, choline tartrate and trichloroacetate.

L-carnitine and its lower alkanoyl derivatives are widely marketed, however, their preparation is exhaustively provided in literature, see for example the patent references in the name of the applicant.

The manufacture of the solutions for peritoneal dialysis according to the present invention requires no more than the commonly known practice owned by the ordinary skilled in the art. An example of useful reference is WO 2004/052269 and related references.

Useful information can be found in the above mentioned patents.

The following description is provided by referring to the preferred member of the carnitine family, namely L-carnitine.

L-carnitine is used in the solution at a concentration ranging from 0.2% w/v to 0.5% w/v. A first preferred range is from 0.02% w/v to 0.45% w/v.

The present invention relates to solutions for peritoneal dialysis. This means to the person skilled in the art that the solution shall be suitable for medical use and contain at least one osmotic agent. Osmotic agent is a solute contained in a concentration sufficient to create an osmotic pressure to replace kidney function by diffusion from the patient's blood across the peritoneal membrane into the solution.

Osmotic agents for peritoneal dialysis are well-known in the art and preferably the agent is selected from the group consisting of: glucose; galactose; xylitol, polyglucose; fructose; sorbitol; glycerol; amino acids, glycerol tripyruvate (tripyruvin), a single peptide, a mixture of peptides, polypeptides, pyruvate compound in the form of a sugar-pyruvate ester, a polyol-pyruvate ester, pyruvate thioester, or a dihydroxyacetone-pyruvate ester. Other osmotic agents, albeit not specifically mentioned here, are not excluded from the present invention.

Osmotic agents for use in peritoneal dialysis are commonly available and a description of these agents can be found in technical literature, for example the above mentioned WO 01/26649, US 5.126.373.

In the present invention, directed to commonly used glucose, it is present in a concentration sufficient to ensure the osmotic effect in conjunction with the concentration of L-carnitine. Usual concentrations known in the art are used, for example from about 1.5% w/v to about 4.5% w/v.

In the present invention L-carnitine is used in a solution comprising glucose and xylitol, the latter at a concentration ranging from 0.5% w/v to 2.5% w/v. A first preferred range is from 0.5% w/v to 1.5% w/v, mostly preferred is 1% w/v.

Glucose and xylitol are present in a concentration sufficient to ensure the osmotic effect in conjunction with the concentration of L-carnitine. In every embodiment of the present invention, when a lower alkanoyl derivative of L-carnitine is used, the concentration will be determined in order to produce an equivalent protective effect, without affecting the osmotic property of the solution.

The same will apply when a salt of a carnitine is used.

The determination of the osmotic effect can be done with any conventional method, for example the one disclosed in the above mentioned WO 01/26649.

For the carrying out the teaching of the present invention, it is not actually necessary that the carnitine be present in an amount sufficient to act as osmotic agent.

The present invention lies on the discovery that a carnitine, in particular L-carnitine, is a protective agent to mesothelium when treated with a solution for peritoneal dialysis, in particular when glucose is the osmotic agent.

Therefore, the advantages of the present invention can be achieved by using low concentrations of carnitine together with the normally used concentrations of glucose in peritoneal dialysis.

However, if it is desired to lower the content of glucose in view of the well known problems, or in case the patient undergoing haemodialysis has already suffered a damage to mesothelium, xylitol can be used as partial substitute of glucose. The present invention is expressed in the embodiment of a ternary solution comprising a carnitine, glucose and xylitol.

The solutions according to the present invention can contain other ingredients conventionally used in the manufacture of solutions for peritoneal dialysis. Reference is made to the general common knowledge of this field.

Another possible embodiment is the use of L-carnitine in the concentration range of from 0.02% w/v to 0.5% w/w in the solutions disclosed in WO 99/07419, wherein a vitamin is selected from the group consisting of folic acid, vitamin B6, thiamine, vitamin B,2, or pharmaceutically acceptable salts thereof

In another convenient embodiment of the present invention, the solution for peritoneal dialysis, further comprises:
120 to about 150 (mEq/L) sodium;
0 to about 110 (mEq/L) chloride;
0 to about 45 (mEq/L) lactate;
0 to about 45 (mEq/L) bicarbonate;
0 to about 4.0 (mEq/L) calcium;
0 to about 4.0 (mEq/L) magnesium.

Conveniently, the solution according to the present invention, can further comprise a substance useful in the treatment of a patient in need of peritoneal dialysis. Such a substance will be determined by the person of ordinary skill in this art, depending on the needs of the patients and his or her conditions, such as the presence of other pathological states of particular needs. Examples of such substance are: vasodilators, diuretics, hormones, vitamins, antioxidants and antifibrotic agents (such as N-substituted 2-(1H) pyridone(s) and/or N-substituted 3-(1H) pyridones.

Another object of the present invention is the use of the solution for peritoneal dialysis herein disclosed in the manufacture of a package for peritoneal dialysis.

A package for peritoneal dialysis is a set of supplies for executing one or more cycles of peritoneal dialysis. A package can contain, for example one or more bags containing the peritoneal dialysis solution of the present invention, even with different formulations, for example for day and/or night cycles. Additional supplies/equipment can be present, such as a disposable connector for executing the peritoneal dialysis cycle. Examples of packages are those commonly marketed.

The following examples further illustrate the invention.

### Example 1

The aim of this example is to compare in vitro and in vivo characteristics of the peritoneal dialysis solutions (PDS) according the present invention with standard bicarbonate glucose peritoneal dialysis solutions.

PDS having the following composition were prepared.

### Glucose with or without carnitine PDS

| | G-2 | GC-2 |
|---|---|---|
| Sodium mmol/l | 134 | 134 |
| Calcium mmol/l | 1.75 | 1.75 |
| Magnesium mmol/l | 0.5 | 0.5 |
| Chlorine mmol/l | 103.5 | 103.5 |
| Bicarbonate mmol/l | 34 | 34 |
| pH | 7.2 | 7.2 |
| Glucose % | 3.86 | 3.86 |
| Carnitine % | - | 0.2 |
| %, (gr/100 ml) | | |

### Xylitol-Glucose PDS

| | XG-1 | XG-2 |
|---|---|---|
| Sodium mmol/l | 134 | 134 |
| Calcium mmol/l | 1.75 | 1.75 |
| Magnesium mmol/l | 0.5 | 0.5 |
| Chlorine mmol/l | 103.5 | 103.5 |
| Bicarbonate mmol/l | 34 | 34 |
| pH | 7.2 | 7.2 |
| Xylitol % | 1.0 | 1.0 |
| Glucose % | 0.36 | 2.86 |
| %, (gr/100 ml) | | |

### Xylitol-Glucose-Carnitine PDS

| | XGC-1 | XGC-2 |
|---|---|---|
| Sodium *mmol*/*l* | 134 | 134 |
| Calcium *mmol*/*l* | 1.75 | 1.75 |
| Magnesium *mmol*/*l* | 0.5 | 0.5 |
| Chlorine *mmol*/*l* | 103.5 | 103.5 |
| Bicarbonate *mmol*/*l* | 34 | 34 |
| pH | 7.2 | 7.2 |
| Xylitol % | 1.0 | 1.0 |
| Glucose % | 0.36 | 2.86 |
| Carnitine % | 0.05 | 0.2 |
| %, (gr/100 ml) | | |

### Studies in vitro

We studied the growth, morphology and function of rabbit peritoneal mesothelial cells cultured in the presence of various PDS containing either glucose alone, glucose and carnitine, xylitol and glucose, or xylitol, glucose and carnitine (see below the exact composition of the various PDS). Glucose was present at two different concentrations (1.36 and 3.86 %, w/v). Carnitine also was present at two different concentrations (0.05 and 0.2 %, w/v). Xylitol concentration, instead, was kept fixed at 1% (w/v).

The biocompatibility of the solutions was evaluated in various tests:
1) growth of mesothelial cell cultures
2) secretion of PLPs and PC by mesothelial cells;
3) secretion of PgE2 by mesothelial cells;
4) LDH release by mesothelial cells.

### Mesothelial cell culture

Cultures of rabbit mesothelial cells were prepared and characterised in the cell pellet as reported previously (see the Di Paolo references above). Confluence cultures of rabbit mesothelial cells were prepared in TC 225 cm² flasks. The culture medium was removed and 20 ml 0.1% trypsin EDTA in PBS A buffer was added to each flask, bathing the layer for a few seconds. After removal of the trypsin mixture, the cells were incubated at 37°C for 5 min to obtain complete detachment of the cells. Then 20 ml medium 199 containing 10% FCS was added to each flask. The cells were characterised as described in Di Paolo.

### Growth of mesothelial cell cultures

The four test PDS solutions were diluted to 20, 40, 60 and 80% with medium 199 containing 10% FCS. Medium without solution was used as control. 3.6 ml of each dilution of the four solutions and pure medium were placed in 102 wells (6 per dilution and 6 controls). 0.4 ml of mesothelial cell suspension (30,000/ml) was sown in each well. Incubation was at 37°C with 5% CO₂ and 95% air. Cell growth was checked daily under the microscope and the number of mesothelial cells was calculated after 7 days of incubation (see the Di Paolo references above).

The following tables 1-3 show the results of the above experiments.

**Table 1**

| | | | | |
|---|---|---|---|---|
| Growth of mesothelial cells (x10³) in culture exposed to PDS containing glucose alone (G-2) and with carnitine (GC-2). PDS were diluted to 20, 40, 60 and 80% with medium 199 containing 10% FCS. Data are expressed as mean ± SD. | | | | |

| % of PDS | | | | |
|---|---|---|---|---|
| | 20 | 40 | 60 | 80 |
| G-2 | 8.11±2.4 | 5.40±1.7 | 1.82+0.8 | 0.86±0.15 |
| GC-2 | 22.2±4.1* | 10.3±2.8** | 4.13±1,8** | 1.04±0.14 |
| *p<0.01;**p<0.05 | | | | |

**Table 2**

| | | | | |
|---|---|---|---|---|
| Growth of mesothelial cells (x10³) in culture exposed to PDS containing glucose and xylitol alone (XG-1) or with carnitine (XGC-1). PDS were diluted to 20, 40, 60 and 80% with medium 199 containing 10% FCS. Data are expressed as mean ± SD. | | | | |

| % of PDS | | | | |
|---|---|---|---|---|
| | 20 | 40 | 60 | 80 |
| XG-1 | 62.2±6.4 | 52.3±6.6 | 15.1±1.8 | 4.51±0.6 |
| XGC-1 | 75.5±6.1** | 68.6±5.8** | 20.4±3,5** | 6.32±0.7* |
| *p<0.01;**p<0.05 | | | | |

**Table 3**

| | | | | |
|---|---|---|---|---|
| Growth of mesothelial cells (x10³) in culture exposed to PDS containing glucose and xylitol alone (XG-2) or with carnitine (XGC-2). PDS were diluted to 20, 40, 60 and 80% with medium 199 containing 10% FCS. Data are expressed as mean ± SD. | | | | |

| % of PDS | | | | |
|---|---|---|---|---|
| | 20 | 40 | 60 | 80 |
| XG-2 | 21.2±2.8 | 14.3±2.7 | 3.51±0.7 | 1.12±0.3 |
| XGC-2 | 32.6±3.1* | 21.4±3.9** | 5.20±1.5 | 1.62±0.7 |
| *p<0.01; **p<0.05 | | | | |

Growth of rabbit mesothelial cells was better in PDS containing carnitine than in the others (Tables 1-3). When carnitine was present in the solution containing only glucose, a statistical significant increase of cell growth was found even in medium containing 60% of PDS (Table 1). In those PDS composed of glucose and xylitol (XG-1 and XG-2), the presence of carnitine (XGC-1 and XGC-2) also led to a statistical significant increase of cell growth. However, a significant effect on mesothelial growth between XG-2 and XGC-2 was only observed in medium containing 20 and 40% of PDS (Table 3), whereas a significant difference between XGC and XGC-1 was observed at all PDS dilution in medium (20, 40, 60 and 80%, see also Table 2). Thus, although the detrimental effect of glucose is clearly dose-dependent (XG-1 vs XG-2), the addition of carnitine is able to attenuate the toxic effect of glucose on mesothelial cell growth irrespective of glucose concentration.

### Substances secreted by cultured mesothelial cells

The culture procedure described for mesothelial cells was repeated in 102 wells with standard medium only. After 6 days of incubation, the supernatant of the cultures was removed and the four PDS, at 100% concentration, were added (6 for each solution and 6 with medium only as controls). After another 6-hours incubation, the supernatant was collected for assay of total phospholipids (PLPs), phosphatidylcholine (PC) (Kit, NEN, Dupont) and PgE2 (RIA kit, New England Nuclear), as indicators of cell functional efficiency, and LDH as indicator of cytotoxicity.

Phospholipids were extracted from supernatant by the method of Folch et al. (Folch J., Lees M. and Stanley HS.; 1957, J. Biol. Chem., 226: 497-509). To enable comparison of data, we used 6 h of incubation, which is the standard time PDS remains in the abdomen in humans.

The following tables 4-6 shows the results of the above experiments.

**Table 4**

| Substances secreted by cultured mesothelial cells exposed to PDS containing glucose alone (G-2) and with carnitine (GC-2). Data are expressed as mean ± SD. | | | | |
|---|---|---|---|---|
| | PC | PLPs | PgE₂ | LDH |
| | µg/ml | µg/ml | mg/ml | U/ml |
| G-2 | 0.45±0.07 | 0.71±0.1 | 0.91±0.09 | 32.7±4.5 |
| GC-2 | 1.03±0.4** | 1.14±0.3** | 1.43±0.4** | 15.3+1.6* |

| | | | | |
|---|---|---|---|---|
| *p<0.01; **p<0.05 | | | | |

**Table 5**

| Substances secreted by cultured mesothelial cells exposed to PDS containing glucose and xylitol alone (XG-1) or with carnitine (XGC-1). Data are expressed as mean ± SD. | | | | |
|---|---|---|---|---|
| | PC | PLPs | PgE₂ | LDH |
| | µg/ml | µg/ml | mg/ml | U/ml |
| XG-1 | 2.21+0.3 | 2.42+0.4 | 2.41+0.3 | 22.2+2.6 |
| XGC-1 | 2.13±0.4 | 2.35±0.3 | 2.56±0.3 | 10.1±0.9* |

| | | | | |
|---|---|---|---|---|
| *p<0.01 | | | | |

**Table 6**

| ubstances secreted by cultured mesothelial cells exposed to PDS containing glucose and xylitol alone (XG-2) or with carnitine (XGC-2). Data are expressed as mean ± SD. | | | | |
|---|---|---|---|---|
| | PC | PLPs | PgE₂ | LDH |
| | µg/ml | µg/ml | mg/ml | U/ml |
| XG-2 | 1.23±0.1 | 1.20±0.2 | 1.30±0.3 | 28.5±3.4 |
| XGC-2 | 1.64±0.3** | 1.78±0.4** | 2.01±0.4** | 18.3±2.5* |

| | | | | |
|---|---|---|---|---|
| *p<0.01; **p<0.05 | | | | |

The highest levels of PLPs and PC secreted by mesothelial cells were found with media containing solutions XG-1 and XGC-1 (Table 4-6), and was indistinguishable from that of controls (data not shown). Instead, the lowest levels of PLPs and PC secreted by mesothelial cells were found with media containing solutions G-2 (Table 4). The presence of carnitine in the latter PDS containing media (GC-2), however, resulted in a statistical significant recovery of mesothelial cells to secrete both PLPs and PC (Table 4). The ability to secrete PC and PLPs was also depressed in the medium containing XG-2, though to a lesser extent than medium containing G-2. The presence of carnitine in the XG2 containing medium (XGC-2) resulted in a significant recovery of both PC and PLPs (Table 6). It is also evident that the more the amount of glucose in PDS (G-2>XG-2>XG>1) the lower the ability of mesothelial cells to secrete PC and PLPs.

It should be noted that elevated carnitine levels may act as a trap of long-acyl-CoAs, which in turn should impair the synthesis of PC and PLPs (PLP synthesis requires several acylation steps, in which the substrate is a of long-acyl-CoA) . Thus, one may consider the above results quite surprising, where the ameliorative action of carnitine on PC and PLPs secretion still remains elusive.

### Secretion of PgE2 by mesothelial cells

Eicosanoids production by mesothelial cells is a valuable index of the dialytic efficiency of the peritoneum. At this regard, we have determined PgE2 in the supernatant of mesothelial cells exposed to different PDS. Among the various PDS tested, the highest PgE2 levels were found in the supernatant of mesothelial cells exposed to XG-1 and XGC-1 (Table 4-6), though the PDS containing carnitine (XGC-1) showed non significant higher levels of PgE2 than PDS without carnitine (XG-1). In addition, no significant differences in PgE2 levels were observed between control and the latter two PDS (data not shown). The lowest PgE2 levels were found in the supernatant of mesothelial cells exposed to G-2. However, the presence of carnitine in this PDS (GC-2) resulted in a remarkable increase of PgE2 recovered. As observed previously for PC and PLPs secretion, the ability to secrete PgE2 was also depressed in the medium containing XG-2, though to a lesser extent than medium containing G-2. The addition of carnitine in the XG2 containing medium (XGC-2) resulted in statistical significant increase of PgE2 levels (Table 6).

### Release of LDH by mesothelial cells

The release of LDH into the supernatant by mesothelial cells represents a useful marker of cell injury. Measurable amounts of LDH were present in the supernatant of mesothelial cells exposed to all the PDS tested, however, those PDS not containing carnitine released statistical significant higher levels of LDH than those containing carnitine (G-2 *vs* GC-2, XG-1 *vs* XGC-1, and GC-2 *vs* XGC-2). In addition, LDH release by mesothelial cells exposed to XGC-1, one of the carnitine-containing PDS, was comparable to control cells (data not shown). Our data also show that the more the amount of glucose in PDS (G-2>XG-2>XG>1) the higher the amount of LDH released by mesothelial cells.

By comparing the effects of PDS containing glucose alone with or without carnitine, and glucose and xylitol with or without carnitine on cultured mesothelial cells, the present inventor arrived at the following findings:
- Mesothelial cells grew much better in PDS containing carnitine than in those PDS not containing it. In the glucose-xylitol PDS containing carnitine, mesothelial growth was higher than in those containing glucose and carnitine. Indeed, the lower the amount of glucose the better the growth. However, the presence of carnitine always showed an improvement of mesothelial growth.
- Phospholipid secretion of mesothelial cells was much higher when carnitine was present in PDS. This is an important index of cell function, as phospholipids are essential for peritoneal physiology. Secretion of phosphatidylcholine, the most active mesothelial surfactant, was indistinguishable between cells cultured in medium with carnitine, (particularly XGC-2) and controls. Significantly lower secretion was observed with medium containing high glucose levels.
- As a general index of cytotoxicity, LDH secretion by mesothelial cells was higher in media with PDS containing glucose alone and glucose plus xylitol than the respective PDS with carnitine.
- Prostaglandin E2 secretion was significantly higher in media containing carnitine.

## Claims

1. A solution for peritoneal dialysis comprising from 0.02% w/v to 0.5% w/v L-carnitine, xylitol and glucose.

2. The solution according to claim 1, wherein carnitine is comprised in the range of 0.02% w/v to 0.45% w/v.

3. The solution according to claim 1 or 2, wherein L-carnitine is substituted or partially substituted by a lower alkanoyl derivative thereof.

4. The solution according to any one of claims 1-3, wherein a pharmaceutically acceptable salt of L-carnitine or a lower alkanoyl derivative thereof is used.

5. The solution according to claim 4, wherein said salt is selected from the group consisting of chloride, bromide, orotate, acid aspartate, acid citrate, magnesium citrate, acid phosphate, fumarate and acid fumarate, magnesium fumarate, lactate, maleate and acid maleate, mucate, acid oxalate, pamoate, acid pamoate, acid sulfate, glucose phosphate, tartrate, acid tartrate, magnesium tartrate, 2-amino ethansulfonate, magnesium 2-amino ethansulfonate, choline tartrate and trichloroacetate.

6. The solution according to any one of claims 1-5, further comprising an additional osmotic agent is selected from the group consisting of: galactose; polyglucose; fructose; sorbitol; glycerol; aminoacids, glycerol tripyruvate (tripyruvin), a single peptide, a mixture of peptides, polypeptides, pyruvate compound in the form of a sugar-pyruvate ester, a polyol-pyruvate ester, pyruvate thioester, or a dihydroxyacetone-pyruvate ester.

7. The solution according to any one of claims 1-5, wherein glucose is comprised in the concentration of 1.5% w/v to 4.5% w/v.

8. The solution according to any one of claims 1-5, wherein xylitol is comprised in a concentration of from 0.5% w/v to 2.5% w/v.

9. The solution according to claim 8, having the following composition
| | |
|---|---|
| Sodium *mmol*/*l* | 134 |
| Calcium *mmol*/*l* | 1.75 |
| Magnesium *mmol*/*l* | 0.5 |
| Chlorine *mmol*/*l* | 103.5 |
| Bicarbonate *mmol*/*l* | 34 |
| pH | 7.2 |
| Xylitol % w/v | 1.0 |
| Glucose % w/v | 0.36 |
| L-Carnitine % w/v | 0.05 |

10. The solution for peritoneal dialysis having the following composition
| | |
|---|---|
| Sodium *mmol*/*l* | 134 |
| Calcium *mmol*/*l* | 1.75 |
| Magnesium *mmol*/*l* | 0.5 |
| Chlorine *mmol*/*l* | 103.5 |
| Bicarbonate *mmol*/*l* | 34 |
| pH | 7.2 |
| Xylitol % w/v | 1.0 |
| Glucose % w/v | 2.86 |
| L-Carnitine % w/v | 0.2 |

11. The solution according to any one of claims 1-8, further comprising:
120 to about 150 (mEq/L) sodium;
0 to about 110 (mEq/L) chloride;
0 to about 45 (mEq/L) lactate;
0 to about 45 (mEq/L) bicarbonate;
0 to about 4.0 (mEq/L) calcium;
0 to about 4.0 (mEq/L) magnesium.

12. The solution according to any one of the preceding claims, further comprising a substance useful in the treatment of a patient in need of peritoneal dialysis.

13. The solution according to claim 12, wherein said substance is selected from the group consisting of: vasodilators, diuretics, hormones, vitamins, antioxidants and antifibrotic agents.

14. The solution according to claim 13, wherein said vitamin is selected from the group consisting of folic acid, vitamin B6, thiamine, vitamin B12, or pharmaceutically acceptable salts thereof.

15. A package for peritoneal dialysis comprising a solution according to any one of claims 1-14.

## Patentansprüche

1. Lösung zur peritonealen Dialyse, umfassend von 0,02 G/V-% bis 0,5 G/V-% L-Carnitin, Xylitol und Glucose.

2. Lösung gemäß Anspruch 1, worin Carnitin im Bereich von 0,02 G/V-% bis 0,45 G/V-% enthalten ist.

3. Lösung gemäß Anspruch 1 oder 2, worin L-Carnitin durch ein niedriges Alkanoyl-Derivat davon ersetzt oder teilweise ersetzt ist.

4. Lösung gemäß irgendeinem der Ansprüche 1 - 3, worin ein pharmazeutisch verträgliches Salz von L-Carnitin oder einem niedrigen Alkanoyl-Derivat davon verwendet wird.

5. Lösung gemäß Anspruch 4, worin das genannte Salz ausgewählt ist aus der Gruppe, bestehend aus Chlorid, Bromid, Orotat, saurem Aspartat, saurem Citrat, Magnesiumcitrat, saurem Phosphat, Fumarat und saurem Fumarat, Magnesiumfumarat, Lactat, Maleat und saurem Maleat, Mucat, saurem Oxalat, Pamoat, saurem Pamoat, saurem Sulfat, Glucosephosphat, Tartrat, saurem Tartrat, Magnesiumtartrat, 2-Aminoethansulfonat, Magnesium-2-aminoethansulfonat, Cholintartrat und Trichloracetat.

6. Lösung gemäß irgendeinem der Ansprüche 1 - 5, weiterhin ein zusätzliches osmotisches Agens umfassend, welches ausgewählt ist aus der Gruppe, bestehend aus: Galactose; Polyglucose; Fructose; Sorbitol; Glycerin; Aminosäuren, Glycerintripyruvat (Tripyruvin), einem einzelnen Peptid, einer Mischung von Peptiden, Polypeptiden, Pyruvatverbindungen, in Form eines Zucker-Pyruvat-Esters, eines Polyol-Pyruvat-Esters, Pyruvat-Thioesters oder eines Dihydroxyaceton-Pyruvat-Esters.

7. Lösung gemäß irgendeinem der Ansprüche 1 - 5, worin Glucose in der Konzentration von 1,5 G/V-% bis 4,5 G/V-% enthalten ist.

8. Lösung gemäß irgendeinem der Ansprüche 1 - 5, worin Xylitol in der Konzentration von 0,5 G/V-% bis 2,5 G/V-% enthalten ist.

9. Lösung gemäß Anspruch 8, welche die folgende 2zusammensetzung besitzt
| | |
|---|---|
| Natrium *mmol*/*l* | 134 |
| Calcium *mmol*/*l* | 1,75 |
| Magnesium *mmol*/*l* | 0,5 |
| Chlor *mmol*/*l* | 103,5 |
| Bicarbonat *mmol*/*l* | 34 |
| pH | 7,2 |
| Xylitol G/V-% | 1,0 |
| Glucose G/V-% | 0,36 |
| L-Carnitin G/V-% | 0,05 |

10. Lösung für die peritoneale Dialyse, welche die folgende Zusammensetzung besitzt
| | |
|---|---|
| Natrium *mmol*/*l* | 134 |
| Calcium *mmol*/*l* | 1,75 |
| Magnesium *mmol*/*l* | 0,5 |
| Chlor *mmol*/*l* | 103,5 |
| Bicarbonat *mmol*/*l* | 34 |
| pH | 7,2 |
| Xylitol G/V-% | 1,0 |
| Glucose G/V-% | 2,86 |
| L-Carnitin G/V-% | 0,2 |

11. Lösung gemäß irgendeinem der Ansprüche 1 - 8, weiterhin umfassend:
120 bis ungefähr 150 (mEq/l) Natrium;
0 bis ungefähr 110 (mEq/l) Chlorid;
0 bis ungefähr 45 (mEq/l) Lactat;
0 bis ungefähr 45 (mEq/l) Bicarbonat;
0 bis ungefähr 4,0 (mEq/l) Calcium;
0 bis ungefähr 4,0 (mEq/l) Magnesium.

12. Lösung gemäß irgendeinem der vorangehenden Ansprüche, weiterhin eine Substanz umfassend, die geeignet ist bei der Behandlung eines Patienten, der einer peritonealen Dialyse bedarf.

13. Lösung gemäß Anspruch 12, worin die genannte Substanz ausgewählt ist aus der Gruppe, bestehend aus:
Vasodilatoren, Diuretika, Hormonen, Vitaminen,
Antioxidanzien und antifibrotischen Agenzien.

14. Lösung gemäß Anspruch 13, worin das genannte Vitamin ausgewählt ist aus der Gruppe, bestehend Folsäure, Vitamin B6, Thiamin, Vitamin B12 oder deren pharmazeutisch verträglichen Salzen.

15. Packung zur peritonealen Dialyse, umfassend eine Lösung gemäß irgendeinem der Ansprüche 1 - 14.

## Revendications

1. Solution de dialyse péritonéale comprenant de 0,02 % poids/volume à 0,5 % poids/volume de L-carnitine, xylitol et glucose.

2. Solution selon la revendication 1, dans laquelle la carnitine est comprise dans la gamme de 0,02 % poids/volume à 0,45 % poids/volume.

3. Solution selon la revendication 1 ou 2, dans laquelle la L-carnitine est substituée ou partiellement substituée par un dérivé alcanoyle inférieur de celle-ci.

4. Solution selon l'une quelconque des revendications 1 à 3, dans laquelle un sel pharmaceutiquement acceptable de L-carnitine ou un dérivé alcanoyle inférieur de celle-ci est utilisé.

5. Solution selon la revendication 4, dans laquelle ledit sel est choisi dans le groupe constitué de chlorure, bromure, orotate, aspartate d'acide, citrate d'acide, citrate de magnésium, phosphate d'acide, fumarate et fumarate d'acide, fumarate de magnésium, lactate, maléate et maléate d'acide, mucate, oxalate d'acide, pamoate, pamoate d'acide, sulfate d'acide, phosphate de glucose, tartrate, tartrate d'acide, tartrate de magnésium, 2-amino-éthane-sulfonate, 2-amino-éthane-sulfonate de magnésium, tartrate de choline et trichloroacétate.

6. Solution selon l'une quelconque des revendications 1 à 5, comprenant en outre un agent osmotique supplémentaire choisi dans le groupe constitué de :
galactose ; polyglucose ; fructose ; sorbitol ;
glycérol ; acides aminés ; tripyruvate de glycérol (tripyruvine), un peptide unique, un mélange de peptides, des polypeptides, un composé de pyruvate sous la forme d'un ester de sucre-pyruvate, un ester de polyol-pyruvate, un thioester de pyruvate ou un ester de dihydroxyacétone-pyruvate.

7. Solution selon l'une quelconque des revendications 1 à 5, dans laquelle le glucose est compris en une concentration de 1,5 % poids/volume à 4,5 % poids/volume.

8. Solution selon l'une quelconque des revendications 1 à 5, dans laquelle le xylitol est compris en une concentration de 0,5 % poids/volume à 2,5 % poids/volume.

9. Solution selon la revendication 8, ayant la composition suivante
| | |
|---|---|
| Sodium mmol/l | 134 |
| Calcium mmol/l | 1,75 |
| Magnésium mmol/l | 0,5 |
| Chlore mmol/l | 103,5 |
| Bicarbonate mmol/l | 34 |
| pH | 7,2 |
| Xylitol % poids/volume | 1,0 |
| Glucose % poids/volume | 0,36 |
| L-Carnitine % poids/volume | 0,05 |

10. Solution de dialyse péritonéale ayant la composition suivante
| | |
|---|---|
| Sodium mmol/l | 134 |
| Calcium mmol/l | 1,75 |
| Magnésium mmol/l | 0,5 |
| Chlore mmol/l | 103,5 |
| Bicarbonate mmol/l | 34 |
| pH | 7,2 |
| Xylitol % poids/volume | 1,0 |
| Glucose % poids/volume | 2,86 |
| L-Carnitine % poids/volume | 0,2 |

11. Solution selon l'une quelconque des revendications 1 à 8, comprenant en outre :
120 à environ 150 (mEq/L) de sodium ;
0 à environ 110 (mEq/L) de chlorure ;
0 à environ 45 (mEq/L) de lactate ;
0 à environ 45 (mEq/L) de bicarbonate ;
0 à environ 4,0 (mEq/L) de calcium ;
0 à environ 4,0 (mEq/L) de magnésium ;

12. Solution selon l'une quelconque des revendications précédentes, comprenant en outre une substance utilisable dans le traitement d'un patient nécessitant une dialyse péritonéale.

13. Solution selon la revendication 12, dans laquelle ladite substance est choisie dans le groupe constitué de : vasodilatateurs, diurétiques, hormones, vitamines, antioxydants et agents antifibrotiques.

14. Solutions selon la revendication 13, dans laquelle ladite vitamine est choisie dans le groupe constitué de l'acide folique, vitamine B6, thiamine, vitamine B12, ou des sels pharmaceutiquement acceptables de ceux-ci.

15. Conditionnement de dialyse péritonéale comprenant une solution selon l'une quelconque des revendications 1 à 14.
